# EUROPEAN PATENT SPECIFICATION

(11) **EP 0 456 188 B1**
(45) Date of publication and mention of the grant of the patent: **30.07.1997**
(21) Application number: 91107405.2
(22) Date of filing: 07.05.1991
(51) Int. Cl.: C07K 14/475, A61K 38/18

(54) **Therapeutic agent for hepatocirrhosis**
Arzneimittel gegen Leberzirrhose
Médicament contre la cirrhose du foie

(30) Priority: 09.05.1990 JP 119030/90
(43) Date of publication of application: 13.11.1991
(73) Proprietor: Nakamura, Toshikazu, Fukuoka-shi, Fukuoka 812 (JP)
(72) Inventor: Nakamura, Toshikazu, Higashi-ku, Fukuoka-shi, Fukuoka 812 (JP)
(74) Representative: von Kreisler, Alek, Dipl.-Chem.

(56) References cited:
- EP-A- 0 412 557
- NATURE. vol. 342, no. 6248, 23 November 1989, LONDON GB pages 440 - 443; NAKAMURA T. et al: "Molecular cloning and expression of human hepatocyte growth factor"
- PATENT ABSTRACTS OF JAPAN vol. 12, no. 230 (C-508)(3077) 29 June 1988, & JP-A- 63 22526 (SHUJI HASHIMOTO) 30 January 1988
- PATENT ABSTRACTS OF JAPAN vol. 11, no. 232 (C-437)(2679) 29 July 1987, & JP-A-62 45530 (Otsuka Pharmaceutical) 27 February 1987
- PATENT ABSTRACTS OF JAPAN vol. 15, no. 60 (C-805)(4588) 13 February 1991, & JP- A-2 288899 (TOYOBO) 28 November 1990
- PATENT ABSTRACTS OF JAPAN vol. 9, no. 175 (C-292)(1898) 19 July 1985, & JP-A- 60 45534 (OTSUKA SEIYAKU) 12 March 1985

## Description

### BACKGROUND OF THE INVENTION

The present invention relates to the use of a hepatocyte growth factor for the preparation of a medicament for suppressing proliferation of hepatic nonparenchymal fibroblasts.

Liver is an organ indispensable for humans, which has a variety of physiological functions such as gluconeogenesis, amino acid metabolism, lipid metabolism, synthesis and secretion of plasma protein, formation and secretion of bile, detoxication, storage of sugar as an energy source, storage of vitamines and so on. The liver develops hepatitis due to virus infection, long-term intake of alcohol or medicaments, or the like, and hepatitis chronically proceeds to hepatocirrhosis. Patients with hepatocirrhosis die with high probability.

At present, the number of patients with hepatocirrhosis in Japan is estimated to be about 400,000, and about 20,000 patients die thereof annually. It is presumed that 80% of the patients suffer from hepatocirrhosis induced by hepatitis caused by B and non-A, non-B virus infections and the most part of the rest 20% suffer from hepatocirrhosis induced by alcoholic hepatitis.

However, there is practically no therapeutic agents for the treatment of hepatocirrhosis. What is being given to the patients is symptomatic therapy by drip infusion and per rectum infusion of vitamins and amino acid fluids to alleviate hypoalimentation and hypoproteinosis observed along with the progress of hepatocirrhosis. Administrations of glycyrrhizin, glutathione, thiopronin, ATP preparations or extracts from animal livers as liver-protecting agents are also employed, albeit with inconclusive effects.

Meanwhile, *in vitro* cultivation of mature parenchymal cells controlling the liver functions had been unattainable for a long time until the present inventor succeeded in partial purification of a protein component on the basis of the finding that mature hepatocytes proliferate extremely well when the particular protein component contained in regenerating hepatic rat sera is added to the medium [Biochem. Biophys. Res. Commun., 122 (No. 3), 1450-1459, 1984], which was named hepatocyte growth factor (hereinafter sometimes referred to as HGF). In addition, the present inventor succeeded in isolating the hepatocyte growth factor from rat platelets [FFBS LETTER, 22 (No. 2), 311, 1987] and determined part of the amino acid sequence (Nature, *342,* 440-443, 1989).

The present inventor further conducted cDNA cloning of human- or rat-originated HGF on the basis of the obtained amino acid sequence of HGF and obtained the hepatocyte growth factor through transfection of said cDNA into animal cells (Nature, 342, 440-443, 1989). EP-A-0 412 557 (Article 54(3),(4) EPC) describes the use of a recombinant human HGF (HW 70-90 kDa) for the preparation of a medicament for the treatment of hepatic diseases as an hepatic cirrhosis-suppressing agent, based on the biological activity of HGF, namely the stimulation of the proliferation of primary cultures of hepatic parenchymal cells or hepatocytes.

Hepatocirrhosis is understood as an ultimate stage of chronic inflammation and fibrosis of a liver. That is, fiber dissepiments are developed throughout the liver, forming innumerable pseudolobules. In other words, it is a state of suppression of hepatocyte growth and abnormal hyperplasia of interstitial bindwebs. Where the fiber dissepiments evolve into pseudolobules, hepatocytes are isolated, preventing maintenance of smooth bloodstream and transportation of substances. Moreover, original vessels become shunts passing through the fiber dissepiments, which gives rise to decrease of effective bloodstream. Such abnormality of bloodstream along with the progress of hepatocirrhosis can be a factor for promoting degeneration of the liver, which encourages vicious circle in hepatocirrhosis.

The main cause of hepatocirrhosis is chronical inflammation over a long period, and hyperplasia of bindwebs is considered to be secondary. For this reason, it is primarily necessary to prevent onset of chronical inflammation. In addition, inhibition of fiber formation by way of suppressing fibroblast proliferation and stimulation of hepatocyte growth are considered to be effective for preventing the transition from chronical inflammation to hepatocirrhosis and progress of hepatocirrhosis, which may eventually enable treatment of even hepatocirrhosis in a considerably advanced stage.

It has been expected that a substance having two physiological activities of specifically stimulating proliferation of hepatocytes and specifically suppressing proliferation of hepatic nonparenchymal fibroblasts can be used as an agent for the treatment of hepatocirrhosis, and provision of such agent is aimed at in the present invention.

### SUMMARY OF THE INVENTION

The present inventor has conducted intensive studies for the purpose of solving the above-mentioned problems, and as a result, found that HGF which has been identified as a hepatocyte growth-stimulating factor also possesses the activity to suppress rat hepatic nonparenchymal fibroblasts in primary culture, which resulted in completion of the invention.

### BRIEF EXPLANATION OF THE DRAWING

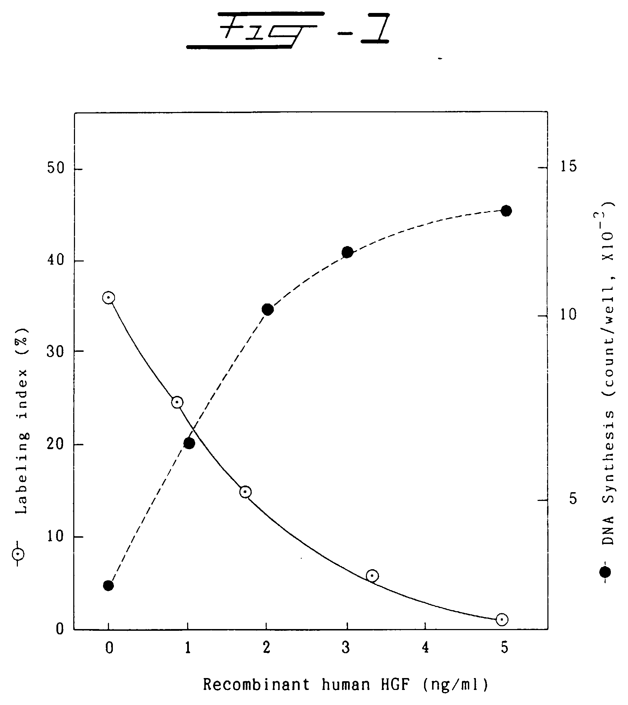

Fig. 1 is a graph showing effects of HGF on hepatocytes in primary culture and on hepatic nonparenchymal fibroblasts in primary culture.

### DETAILED DESCRIPTION OF THE INVENTION

The present invention relates to the use of a hepatocyte growth factor for the preparation of a medicament for suppressing proliferation of hepatic nonparenchymal fibroblasts, wherein the hepatocyte growth factor is a glycoprotein having a molecular weight of 70.000 to 90.000 by nonreduction polyacrylamide gel electrophoresis.

Under reducing conditions, the HGF used in the present invention takes the heterodimer structure comprising α-chain of 60,000 to 75,000 molecular weight and β-chain of 30,000 to 40,000 molecular weight. Based on the amino acid sequence of human HGF and rat HGF, it is speculated that the α-chain has a special sequence called kringle structure as can be seen in plasminogen and plasmin, and the sequence in the β-chain resembles the serine · protease domain such as kallikrein and coagulation factor XII. Since HGF shows affinity to concanavalin A, it is a glycoprotein having a sugar chain therein.

HGF can be obtained by various methods. For example, it can be obtained from organs such as liver, spleen, lung, bone marrow, brain, kidney and placenta, blood cells such as platelet and leukocyte and plasma (including serum) of mammals such as cow by extraction and purification. It is also possible to obtain HGF by cultivation of primary culture cells and strain cells capable of HGF production, followed by separation and purification from the culture. Needless to say, HGF can be obtained by genetic engineering comprising isolation of the HGF gene, transformation of suitable host cells and cultivation of the transformants obtained, followed by isolation-purification of the objective recombinant hepatocyte growth factor from the culture (See Nature, *342*, 440-443, 1989).

Recombinant human HGF is prepared by, for example,;
(1) mRNA or genomic DNA is isolated from animal tissues such as rat hepatocytes or rat megakaryocytes, from which cDNA libraries or genomic DNA libraries are prepared;
(2) desired cDNAs or genomic DNAs are prepared from isolated clones by screening the above-mentioned animal- (e.g. rat) originated cDNA libraries or genomic DNA libraries for isolation of the cDNA or genomic DNA of HGF of an animal such as rat with synthetic oligonucleotide probes or antibodies, and objective human-originated HGF cDNAs are extracted from the isolated clones by screening the cDNA libraries prepared from mRNA of human organs or blood cells with the rat-originated HGF cDNAs or genomic DNAs as the probe. Also, human-originated HGF cDNAs can be prepared from the isolated clones by screening the cDNA libraries constructed from mRNA extracted from human organs or blood cells. The screening of the cDNA library mentioned can be carried out by using as the probe, the human HGF cDNAs or the human HGF genomic DNAs synthesized on the basis of the DNA sequence determined by the present inventor or the human or animal HGF amino acid sequence, or by using antibodies against human or animal HGF;
(3) cDNA fragments encoding the human HGF are cleaved from the human-originated HGF cDNAs with restriction enzymes and integrated in the expression vectors;
(4) By the obtained recombinant expression vectors, host cells are transformed to obtain transformants;
(5) By cultivating the transformants, the human HGF can be obtained from the culture supernatant.

Besides, the DNAs containing the nucleotide sequences which code for the human HGF can be obtained from the recombinant expression vectors in the transformed cells by way of treatment with restriction enzymes.

The respective processes are in detail described below.

### (1) Isolation of mRNA and preparation of cDNA library:

mRNA encoding animal (e.g. rat) or human HGF can be obtained respectively from organs such as liver, kidney, spleen, lung, brain, bone marrow and placenta or blood cells such as leukocyte or megakaryocyte of animals such as rat or human. For example, in accordance with the J. M. Chirgvin et al method as described in Biochemistry, *18*, 5294 (1979), said mRNA can be purified by subjecting RNA obtained from the guanidine thiocyanate lysate of animals (e.g. rat) or human organs or blood cells to liquid chromatography of oligo (dT) cellulose column.

Also, various mRNAs of animal cells or tissues such as those of human liver, brain, placenta, leukocyte and so on are available in the market, and the products of Clontech Lab. can be used.

cDNA libraries can be constructed by synthesizing cDNAs using a reverse transcriptase with the above-mentioned mRNA as a template or using polymerase chain reaction (PCR) method, in accordance with, for example, the H. Okayama et al method (Mol. Cell. Biol., *2*, 161. 1982 and Mol. Cell. Biol., *3*, 280, 1983), the U. Gubler et al method (Gene, *25*, 263, 1983) or the M.A. Frohman method (Proc. Natl. Acad. Sci. USA, *85,* 8998, 1988) and inserting said cDNAs into plasmids or phage DNAs. Examples of the plasmid vectors into which the cDNAs are inserted include *Escherichia coli*-originated pBR322, pUC18 and pUC19 (Toyobo) and *Bacillus* s*ubtilis*-originated pUB110 (Sigma). As the phage vectors into which the cDNAs are inserted, mention is made of λ gt10 and λ gt11 (Toyobo). Besides the above-mentioned examples of the vectors, any vector can be used as long as it is capable of replication and amplification in host cells.

As the methods in which the cDNAs synthesized from mRNA as the template are inserted into plasmids or phage DNAs to produce the cDNA libraries, mention can be made of, for example, the T. Maniatis's method (Molecular Cloning, Cold Spring Harbor Laboratory, 1982, p.239) or the T. V. Hyunh et al method (DNA Cloning : A Practical Approach, *1*, 49, 1985). Also, various kinds of cDNA libraries are available in the market like mRNAs and can e.g. be purchased from Clontech Corp.

### (2) Cloning of cDNA library:

The recombinant expression vectors of plasmids, phage DNAs or so on obtained as cDNA libraries can be maintained in suitable host cells such as *Escherichia coli.* Examples of the *Escherichia coli* as the host cell include *Escherichia coli* NM514, C600 (Stratagene), NM522, JM101 (Pharmacia) and so on. Using the calcium chloride method, the calcium chloride-rubidium chloride method or another method in the case where plasmids are used as the cDNA vector and using the *in vitro* packaging method in the case where phage DNAs are used as the cDNA vector, the recombinant expression vectors can be maintained in host cells which are in advance proliferated (Molecular Cloning, Cold Spring Harbor Laboratory, 1982, p.249).

Oligonucleotides encoding a portion of the amino acid sequences of the hepatocyte growth factors of animal such as rat or human are synthesized. From the thus-obtained transformants, cDNA clones can be screened by using the above-mentioned oligonucleotides labeled with ³²P as a probe by the colony-hybridization method (Gene, *10,* 63, 1980), the plaque hybridization method (Science, *196*, 180, 1977) or another method. The cloning can be conducted also by the enzyme antibody method (DNA Cloning: A Practical Approach, *1*, 49, 1985) with antibodies against the objective polypeptides to give the cDNA clones. The thus-cloned transformants contain the cDNAs having the nucleotide sequences which encode the entire amino acid sequence or a portion of the amino acid sequence of animal- (e.g. rat) or human-originated HGF.

The recombinant DNAs of the plasmid or phage are isolated from the transformants in accordance with the conventional method (Molecular Cloning, Cold Spring Harbor Laboratory, New York, 1982), wherefrom directly as it is or after digested with restriction enzymes, the cDNA nucleotide sequence is determined. The cDNA libraries prepared from mRNA originated from human organs or blood cells are screened in the same manner with the first-obtained animal- (e.g. rat) or human-originated cDNAs as the probe. The cDNA sequence of animal- such as rat or human-originated HGF can be determined by the Maxam and Gilbert's chemical method (Proc. Natl. Acad. Sci. USA, *74*, 560, 1977) or the dideoxy method by Sanger (Proc. Natl. Acad. Sci. USA, *74,* 5463, 1977). Furthermore, if desired, another cDNAs are synthesized newly from the above-mentioned mRNA by the primer-extension method (Proc. Natl. Acad. Sci. USA, *76*, 731, 1979) using as the primer a portion of the cDNA whose sequence is determined or a synthesized DNA which is a pert of the cDNA, and the cloning of the recombinant DNAs of plasmids, phages or so on containing the cDNAs which can be ligated with the cDNA obtained already from the cDNA library can be performed in the same manner as mentioned above. These steps of primer-extension and cloning can be repeated multiple times, if necessary.

### (3) Construction of recombinant expression vector for human HGF:

Recombinant expression vectors can be prepared by digesting restriction enzymes cDNAs from several kinds of recombinant vectors of plasmids, phages or so on containing the cDNAs which encode the entire amino acid sequence or portion thereof of human HGF and ligating the cDNAs with the downstream of promoter region of vectors suitable for expression of the human HGF with DNA ligase.

More specifically, for the purpose of permitting the human HGF to express efficiently, the recombinant expression vector is constructed, if desired, so that it may contain (1) a promotor, (2) a ribosome binding site, (3) an initiation codon, (4) a DNA containing the nucleotide sequence which encodes the human HGF, (5) a termination codon and (6) a terminator in order in the downstream direction of transcription.

As the DNA vectors to be used in the present invention, mention can be made of, for example, *Escherichia coli*-originated plasmid pBR322, pUC18 (Toyobo), Bacillus, subtilis-originated plasmid pUB110 (Sigma), yeast-originated plasmid pRB15 (ATCC37062), bacteriophage λ gt10, λ gt11, (Stratagene Corp.), Virus SV40 (BRL Corp.), BPV (ATCC VR-703) and retrovirus gene-originated vectors. Besides the above vectors, any DNA vector can be used as long as it is a vector capable of replication and amplification in a host. Particularly, vectors orginated from a virus gene such as SV40 are preferable in order to allow the human HGF to express easily.

For example, a recombinant expression vector in which the above-mentioned cloned DNA encoding the human HGF is ligated with the late promoter region of SV40 DNA can be introduced into a simian cell strain called COS cell (Cell, *23*, 175, 1981) for expression.

With regard to the promoter and terminator, any one can be used as long as it corresponds to the host used for expression of the nucleotide sequences which code for the objective human HGF. For example, there can be mentioned trp promoter, lac promoter and so on as the promoter for the host of *Escherichia coli,* SP01 promoter, SP02 promoter and so on for the host of *Bacillus subtilis,* GAP promoter, PGK promoter and so on for the host of yeast and virus-originated SV40 promoter, HSV1 TK promoter, and metallothionein promoter for the host of animal cells such as mouse fibroblast and Chinese-hamster ovary cell. As the terminator, there can be mentioned, for example,trp terminator, lpp terminator and so on for the host of *Escherichia coli,* amyF terminator for the host of *Bacillus subtilis,* CYC1 terminator for the host of yeast and SV40 terminator, HSV1 TK terminator and so on for the host of animal cells. These promoters and teminators can be used suitably in combination depending on the host used.

There is no particular limitation to the DNA containing base sequences which code for the human HGF as long as the polypeptides prepared from DNA by transcription and translation possess the hepatocyte growth activity, and DNAs having a nucleotide sequence in which said nucleotide sequence is partially substituted, deleted and/or inserted may be used. The translation intiation codon of the DNAs containing the nucleotide sequences which code for the human HGF may have ATG, and the translation termination codon may have TAA, TGA or TAG. If desired, more than one termination codon may exist in combination with an initiation condon. Initiation codon and termination codons are not limited. It is preferable that the vectors contain at a suitable position one or more ampicillin-resistance gene(s), neomycine-resistance gene(s), DHFR gene(s) and so on which can be a selection marker for the hosts which are transformed by the recombinant expression vectors.

### (4) Transformation of host cell and cultivation thereof:

The thus-constructed recombinant expression vectors for the human HGF are introduced into host cells to yield transformants by the competent cell method (J. Mol. Biol., *53*, 154, 1970), the protoplast method (Proc. Natl. Acad. Sci. USA, *75*, 1929, 1978), the calcium phosphate method (Science, *221,* 551, 1983), the DEAE dextran method (Science, *215*, 166, 1982), the electroporation method (Proc. Natl. Acad. Sci. USA, *81,* 7161, 1984), the *in vitro* packaging method (Proc. Natl. Acad. Sci. USA, *72*, 581, 1975), the virus vector method (Cell, *37*, 1053, 1984) or the microinjection method (Exp. Cell. Res., *153*, 347, 1984). Herein, use can be made of as the host cell not only Eschelichia coli as mentioned above but also *Bacillus subtilis,* yeasts, and animal cells. Particularly, it is preferred to use mammalian host cells such as mouse fibroblast C127 (J. Virol., *26*, 291, 1978) and chinese hamster ovary cell CHO (Proc. Natl. Acad. Sci. USA, *77*, 4216, 1980).

The thus-obtained transformants are cultivated in a culture medium suitable for the host to produce the objective recombinant human HGF. In the culture medium, there may be contained carbon sources, nitrogen sources, inorganic substances, vitamine, sera and medicaments which are necessary for growth of the transformants. Examples of the culture medium include LB medium (Nissui Pharmaceutical) and M9 medium (J. Exp. Mol. Genet., Cold Spring Harbor Laboratory, New York, 1972, p.431) in the case where the host of the transformant is *Escherichia coli ;* YEPD medium (Genetic Engineering, vol. 1, Plenum Press, New York, 1979, p. 117) in the case where the host is yeast; and MEM medium, DMEM medium and RPM11640 medium (Nissui Pharmaceutical) containing fetal bovine serum in a proportion of not more than 20%, in the case where the host is an animal cell. The cultivation of the transformants is conducted usually at 20°C - 45°C at pH 5-8, while if necessary aeration and stirring is conducted. When the host is an adherent animal cell, carriers are used such as glass beads, collagen beads or acetyl cellulose hollow fibres. The cultivation can be conducted in a medium of any other medium composition under any other conditions so long as the transformant grows. Thus, said medium composition and cultivation conditions are not limited.

### (5) Purification of human HGF:

The recombinant human HGF produced thus in the culture supernatant of the transformants or in the transformants can be separated and purified by known methods such as salt precipitation, solvent precipitation, dialysis, ultrafiltration, gel electrophoresis, gel filtration chromatography, ion exchange chromatography, reverse-phase chromatography and affinity chromatography in combination. Particularly, preferred and effective purification methods are a combination of salt precipitation with ammonium sulfate, S-Sepharose ion exchange chromatography, heparin-Sepharose affinity chromatography and phenyl-Sepharose reverse-phase chromatography and a combination of salt precipitation with ammonium sulfate, S-Sepharose ion exchange chromatography and anti HGF antibody-Sepharose affinity chromatography.

The recombinant human HGF obtained by the above-mentioned methods exhibits an excellent growth promoting activity for hepatocytes in the same manner as with a rat liver- or rat platelet-originated HGF.

It is also possible to extract, isolate and purify HGF from animal tissues including those of humans and cultured animal cells, and methods therefor include conventional extraction, isolation and purification for proteins. For example, organic solvent precipitation with ethanol or acetone, salt precipitation with ammonium sulfate, dialysis, ultrafiltration, ion-exchange chromatography, gel filtration chromatography, reverse-phase chromatography, hydrophobic chromatography and affinity chromatography may be used in combination. Particularly preferred are combinations of S-Sepharose chromatography, heparin-Sepharose chromatography, phenyl-Sepharose chromatography, antibody affinity chromatography, C4 reverse-phase chromatography and pigment affinity chromatography, which are effective methods of isolation and purification.

Any HGF can be used for the present invention as long as it has sufficient fibroblasts growth-inhibitory activity, even if part of its amino acid sequence is deleted or substituted, or other amino acid sequence is partly inserted, or sugar is deleted or substituted in the same manner.

The HGF which is an active ingredient in the medicament of the present invention shows excellent activities for suppression of fibroblast growth in mammals such as human, cow, horse, rat and sheep.

The medicament of the invention is generally formed into injections containing HGF solely or combinedly with carriers known per se. For example, injections can be prepared by dissolving HGF in suitable buffers, followed by sterilization by filtration through a filter.

The medicament of the invention may contain other additives such as stabilizers, excipients, dissolution-promotors, adsorption-preventors and antioxidants, and examples thereof include, for example, sugars such as mannitol and glucose, amino acids such as glycine, alanine, lysine and arginine, proteins such as albumin, alcohols such as ethylene glycol and glycerol, hydrophilic polymers such as polyethylene glycol, inorganic salts such as NaCl, organic salts such as sodium citrate, surfactants such as Polysorbate 80 and reducing agents containing sulfur, which may be used alone or in combination.

The liquid preparations are preferably stored by cyropreservation or after removal of water content by freeze-drying or vacuum drying. An aqueous solution containing HGF may be subjected to salt precipitation or solvent precipitation for precipitation of the factor, after which the obtained precipitate is dried and stored.

The medicament of the invention can be generally administered intravenously, intra-arterially or subcutaneously. The dose amount ranges from 0.01 mg to 100 mg by the amount of HGF, which can be administered singly or in several times divided doses.

The medicament of the invention contain HGF as an active ingredient. The HGF not only suppresses fiber formation by the fibroblast proliferation-inhibitory activity but simultaneously stimulates hepatocyte proliferation, exhibiting two physiological activities of specific stimulation of hepatocyte proliferation and specific inhibition of hepatic nonparenchymal fibroblast proliferation, and thus, is considered to be effective for preventing the transition from chronic hepatitis to hepatocirrhosis and progress of hepatocirrhosis, as well as for the treatment of considerably advanced hepatocirrhosis. Thus, HGF is highly useful as a therapeutic agent for hepatocirrhosis for which there have been no dependable treatment methods, and also for hepatic diseases such as hepatitis.

The present invention is hereinbelow described in detail by illustrating working examples, reference examples and experimental examples.

### Reference Example 1

### [Assay of HGF activity]

HGF activity was assayed in the following manner in accordance with the method described in Proc. Natl. Acad. Sci. USA, *80*, 7229 (1983). Hepatocytes were isolated and purified from Wister rats by the collagenase reflux method. The obtained rat hepatocytes were suspended in the Williams E medium (Flow-Laboratory Corp.) to which 5% fetal bovine sera, 2 × 10⁻⁹ M insulin and 2 × 10⁻⁹ M dexamethasone had been added, and the suspension was sown on a 24-well multiplate in the concentration of 1.25 × 10⁵ cells per well. After the cells were cultured in the presence of 5% CO₂, 30% O₂ and 65% N₂ at 37°C for 20 hours, the medium was replaced with Williams E medium containing 0.1 µg/ml of aprotinin, and at the same time, the prescribed amount of test samples were added. Fifteen hours later, 15 µCi/ml of ¹²⁵I-deoxyuridine was added thereto (10 µl/well). To the control, 5 µg/ml of aphidicolin was added 15 minutes before the addition of ¹²⁵I-deoxyuridine. The cells were cultured for further 6 hours and labeled with ¹²⁵I. The cells were washed twice with PBS at pH 7.4 and then immobilized with a cold 10% trichloroacetic acid (TCA) solution. The cells were solubilized with 1N NaOH in an amount of 0.5 ml per well, and the radioactivity was assayed by a gamma counter. After the assay of radioactivity, a portion of the sample was measured for the amount of the protein in accordance with the Lowry method (J. Biol. Chem., *193*, 265, 1951). The amount of ¹²⁵I incorporated into the hepatocytes with the addition of the test sample was measured as the difference in count from that of the control, from which the amount per 1 mg of the rat hepatocyte protein was estimated and taken as the DNA synthetic activity (dpm/mg protein). The HGF activity of the test sample was indicated on the basis of the definition that the activity corresponding to the 50% synthetic activity of the hepatocyte DNA in the same test with the use of 10 ng/ml of epithelial cell growth factor (EGF) being 1 unit.

### Reference Example 2

### [Assay of hepatic nonparenchymal fibroblast proliferation-inhibitory activity]

A crude cell dispersion was prepared by perfusing liver from young mature rat (weighing about 180 g) with collagenase, which was then subjected to low speed centrifugation at 50 × g for 1 minute to isolate the supernatant from hepatocytes precipitated.

The supernatant was subjected to centrifugation at 100 × g for 3 minutes to completely precipitate parenchymal hepatocytes, followed by centrifugation at 350 × g for 3 minutes to precipitate nonparenchymal hepatocytes. The thus-obtained nonparenchymal hepatocytes containing practically no parenchymal hepatocytes were suspended in an RPMI 1640 medium containing 10% bovine serum at a concentration of about 260,000 cells/ml, and cultivated in a 12-well multiplate at 1 ml per well. After 3 days' cultivation, the medium was replaced with a fresh RPMI 1640 medium containing 10% fetal calf serum and cultivation was continued. On day 7 from initiation of the cultivation, human or rat liver-originated HGF was added at a concentration of from 1 ng to 5 ng/ml. Twenty-four hours later, 2.5 µCi of [³H]-thymidine was added to each well, followed by further cultivation for 12 hours. The medium was removed and cells were washed well with PBS. A gender solution (1 ml) was added to each well to immobilize the cells. Twenty minutes later, the gender solution was removed, and the cells were washed with PBS and coated with an X-ray photosensitive emulsion [emulsion type NR-M2 (Konishiroku Corp.)] in a darkroom. After 6 days' exposure at 4°C, the cells were fixed and developed to yield an autoradiogram. After developing and fixation, cytoplasms were stained with eosin and photographed. Labeling index was estimated by counting nonparenchymal hepatocytes whose nuclei were labeled.

### Reference Example 3

### [Production of hepatocyte growth factor from liver]

Carbon tetrachloride (0.2% body weight of rat) was intraperitoneally administered, and 30 hours later, the rat liver was excised. The liver was pulverized by a whirling blender, after which it was centrifuged at 10,000 rpm for 20 minutes with a Hitachi 20 PR-52 cooling centrifuge to give the supernatant. The supernatant was dialyzed with a mixed solution of 50 mM tris hydrochloric acid (pH 8.5), 0.15 M NaCl, 10 mM HEPES, 2 mM CaCl₂ and 0.01% Tween 80 at 4°C for a whole day. The dialyzed solution was poured onto an S-Sepharose (FF, Pharmacia) column equilibrated with dialysing fluid, and after washing, it was eluted with the concentration gradient of NaCl. The hepatocyte growth factor was eluted near the NaCl concentration of 0.7 M. The hepatocyte growth facter was then purified by Blue Tris acryl M (IBF Corp.) chromatography. Elution was conducted with the concentration gradient of arginine, and the hepatocyte growth factor was eluted at an arginine concentration of near 0.25 M. The obtained fraction was then purified by heparin-Sepharose (Pharmacia) chromatography. Elution was conducted with the concentration gradient of NaCl, and the hepatocyte growth factor was eluted at an NaCl concentration of about 1 M, which was then purified by phenyl 5PW (Toso Corp.) chromatography. Elution was conducted with the concentration decrease gradient of NaCl and the concentration increase gradient of ethylene glycol, and the hepatocyte growth factor was obtained by 10 µg per livers from 100 rats. The purified hepatocyte growth factor showed a band of 70,000 to 90,000 molecular weight under nonreducing conditions and after reduction, it showed two bands of α-chain of about 70,000 molecular weight and β-chain of about 30,000 molecular weight by SDS-PAGE electrophoresis. To the obtained hepatocyte growth factor was added 0.25% BSA (bovine serum albumin), which was then dialyzed with PBS and used for experiment.

### Reference Example 4

### [Production of human hepatocyte growth factor using C127 cells as a host by genetic recombination]

Mouse C127 cells transformed by a gene coding for the amino acid sequence of human hepatocyte growth factor were cultivated, and human hepatocyte growth factor was obtained from the culture supernatant thereof. The processes are described in the following.

Clone HAC19 and HBC25 coding for the amino acid sequence of human hepatocyte growth factor were obtained by screening cDNA libraries constructed from human liver mRNAs.

DNAs from the HAC19 and the HBC25 were digested with BamHI and ScaI, and ScaI and PstI, respectively. The thus-obtained two DNA fragments were ligated with Blue Script KSII at BamHI and PstI sites to obtain pBS[hHGFII] (FERM P-11050 deposited at the Fermentation Research Institute, Agency of Industrial Science and Technology, Ministry of International Trade and Industry, Japan). The pBS[hHGFII] was digested with XbaI, SalI and NaeI, and given blunt ends by T4 DNA polymerase, after which an about 3 Kb DNA fragment coding for human hepatocyte growth factor was inserted at EcoRV site of an expression vector pBPMT prepared with bovine papilloma virus DNA as a vector, to give pBPMT[hHGFII]. The thus-obtained hepatocyte growth factor expression vector pBPMT[hHGFII] was used to transform mouse C127 cells by the calcium phosphate method. Selection of the transformants was conducted by growth thereof in a medium containing G418. The cell line BPH89 showing high producibility of a hepatocyte growth factor was selected from among the obtained transformants. After the BPH89 cells were grown in a medium supplemented with fetal calf serum, the medium was replaced every 2 days to permit production of a hepatocyte growth factor. The objective hepatocyte growth factor was purified from the culture medium by a modification of the purification method as described in Reference Example 3. It was confirmed that the purified hepatocyte growth factor showed a single band of about 80,000 daltons molecular weight under nonreducing conditions and two bands of α-chain of about 70,000 daltons molecular weight and β-chain of about 30,000 daltons molecular weight under reducing conditions by SDS polyacrylamide gel electrophoresis. To the obtained hepatocyte growth factor was added 0.25% BSA and after dialysis with PBS, it was used for experiment.

### Experimental Example

### [Effect of HGF on hepatocytes in primary culture and on hepatic nonparenchymal fibroblasts in primary culture]

The purified human recombinant hepatocyte growth factor as prepared according to Reference Example 4 was added to primary mature rat hepatocyte culture so that the concentration became 1-5 ng/ml according to the method as described in Reference Example 1, and DNA synthesis was measured. Further, the factor was added to rat nonparenchymal hepatocytes at 1-5 ng/ml as described in Reference Example 2, and labeling index was calculated. The results are summarized in a graph as Fig. 1. The recombinant human hepatocyte growth factor promoted growth of hepatocytes dose-dependently, whereas markedly inhibited growth of nonparenchymal hepatocytes. That is, the factor acts completely reversely on parenchymal cells and nonparenchymal cells constituting a liver.

### Example 1

An aqueous solution is prepared aseptically by adding 1 mg of a hepatocyte growth factor and 100 mg of human serum albumin to 100 ml of 0.02 M phosphate buffer (pH 7.4) containing 0.15 M NaCl and 0.01% Polysorbate 80, and filled in a vial at 1 ml per vial, followed by lyophilization and sealing. Injectable distilled water is filled in an ampoule at 1 ml each for dissolution.

### Example 2

An aqueous solution is prepared aseptically by adding 1 mg of a hepatocyte growth factor and 100 mg of human serum albumin to 100 ml of 0.02 M phosphate buffer (pH 7.4) containing 0.15 M NaCl and 0.01% Polysorbate 80, and aseptically filled in an ampoule at 1 ml per ampoule, followed by melt-sealing.

### Example 3

A solution is prepared aseptically by adding 1 mg of a hepatocyte growth factor, 1 g of mannitol and 10 mg of Polysorbate 80 to 100 ml of physiological saline and filled in a vial at 1 ml per vial, which is then lyophilized and sealed.

### Example 4

An aqueous solution containing 1 part by weight of a hepatocyte growth factor, 50 parts by weight of human serum albumin and 100,000 parts by weight of injectable distilled water is prepared aseptically and filled in a vial, which is then lyophilized and sealed.

### Example 5

A solution is prepared aseptically by adding 1 mg of a hepatocyte growth factor, 10 mg of Polysorbate 80, 2 g of glycine and 2 g of sorbitol to 100 ml of injectable distilled water, and filled in a vial at 1 ml per vial, which is then lyophilized and sealed.

## Claims

1. Use of a hepatocyte growth factor for the preparation of a medicament for suppressing proliferation of hepatic nonparenchymal fibroblasts, wherein the hepatocyte growth factor is a glycoprotein having a molecular weight of 70.000 to 90.000 by nonreduction polyacrylamide gel electrophoresis.

2. Use as claimed in claim 1, wherein the hepatocyte growth factor is originated from animal tissues including those of human.

3. Use as claimed in claim 1, wherein the hepatocyte growth factor takes the heterodimer structure comprising an α-chain of 60.000 to 75.000 molecular weight and a β-chain of 30.000 to 40.000 molecular weight under reducing conditions.

4. Use as claimed in claim 1, wherein the medicament contains a therapeutically effective amount of the hepatocyte growth factor in admixture with a pharmaceutically acceptable carrier.

5. Use as claimed in claim 1, wherein the medicament is suitable for parenteral administration.

## Patentansprüche

1. Verwendung eines Hepatocyten-Wachstumsfaktors zur Herstellung eines Medikaments zur Unterdrückung der Proliferation nichtparenchymaler Leberfibroblasten, wobei der Hepatocyten-Wachstumsfaktor ein Glycoprotein mit einem durch nichtreduzierende Polyacrylamidgel-Elektrophorese bestimmten Molekulargewicht von 70 000 bis 90 000 ist.

2. Verwendung gemäß Anspruch 1, wobei der Hepatocyten-Wachstumsfaktor von tierischen Geweben einschließlich solchen des Menschen abgeleitet ist.

3. Verwendung gemäß Anspruch 1, wobei der Hepatocyten-Wachstumsfaktor die Heterodimer-Struktur annimmt, die unter reduzierenden Bedingungen eine α-Kette mit einem Molekulargewicht von 60 000 bis 75 000 und eine β-Kette mit einem Molekulargewicht von 30 000 bis 40 000 umfaßt.

4. Verwendung gemäß Anspruch 1, wobei das Medikament eine therapeutisch wirksame Menge des Hepatocyten-Wachstumsfaktors im Gemisch mit einem pharmazeutisch annehmbaren Träger enthält.

5. Verwendung gemäß Anspruch 1, wobei das Medikament für die parenterale Verabreichung geeignet ist.

## Revendications

1. Utilisation d'un facteur de croissance hépatocyte pour la préparation d'un médicament pour supprimer la prolifération de fibroplastes non parenchymateux hépatiques, dans laquelle le facteur de croissance hépatocyte est une glycoprotéine ayant un poids moléculaire de 70.000 à 90.000 par électrophorèse de gel de polyacrylamide non réductrice.

2. Utilisation selon la revendication 1, dans laquelle le facteur de croissance hépatocyte provient de tissus animaux y compris ceux de l'homme.

3. Utilisation selon la revendication 1, dans laquelle le facteur de croissance hépatocyte a la structure hétèrodimère comprenant une chaîne α de poids moléculaire de 60.000 à 75.000 et une chaîne β de poids moléculaire de 30.000 à 40.000 sous des conditions réductrices.

4. Utilisation selon la revendication 1, dans laquelle le médicament contient une quantité efficace du point de vue thérapeutique du facteur de croissance hépatocyte en mélange avec un véhicule acceptable du point de vue pharmaceutique.

5. Utilisation selon la revendication 1, dans laquelle le médicament est approprié pour l'administration par voie parentérale.
